# EUROPÄISCHE PATENTSCHRIFT

(11) **EP 2 760 385 B1**
(45) Veröffentlichungstag und Bekanntmachung des Hinweises auf die Patenterteilung: **16.09.2015**
(21) Anmeldenummer: 12775428.1
(22) Anmeldetag: 17.09.2012
(51) Int. Cl.: A61F 5/058

(54) **EINRICHTUNG ZUR STÜTZUNG UND STABILISIERUNG EINES VERLETZTEN**
DEVICE FOR SUPPORTING AND STABILIZING AN INJURED PERSON
DISPOSITIF DE SOUTIEN ET DE STABILISATION D'UN BLESSÉ

(30) Priorität: 27.09.2011 AT 13952011
(43) Veröffentlichungstag der Anmeldung: 06.08.2014
(73) Patentinhaber: Kohlbrat & Bunz Gesellschaft m.b.H, 5550 Radstadt (AT)
(72) Erfinder: RUGFELT, Hakan, 239 41 Falsterbo (SE)
(74) Vertreter: Torggler, Paul Norbert
(86) Internationale Anmeldenummer: PCT/AT2012/000239
(87) Internationale Veröffentlichungsnummer: WO 2013/044277

(56) Entgegenhaltungen:
- WO-A1-01/30280
- WO-A1-2004/004608
- WO-A2-99/16392

## Beschreibung

Die Erfindung betrifft eine Einrichtung zur Stützung und Stabilisierung eines Verletzten, mit einem flexiblen, am Verletzten bzw. an einem verletzten Körperteil festlegbaren Folienelement, das zwei Folien und einen Granulat enthaltenden, evakuierbaren Innenraum aufweist, wobei die beiden Folien entlang des Randes und an Stellen im Inneren im Wesentlichen punktförmig so miteinander verbunden sind, dass Kanäle parallel zu mindestens einem Seitenrand verbleiben.

Derartige auch als Vakuummatratzen und Vakuumschienen bezeichnete Stabilisierungseinrichtungen für Transport und Immobilisierung des Verletzten weisen eine Hülle aus einer luftdichten Kunststofffolie und eine Füllung aus einem Kunststoffgranulat, insbesondere aus geschäumten Polystyrolkugeln, auf und können nach der Anpassung und Fixierung an einem ruhig zu stellenden Körperteil mittels einer Saugpumpe evakuiert werden. Dies führt zu einer dichten Packung des lose eingefüllten Granulats und somit zu einer Versteifung des flexiblen Elements, das auf diese Weise eine im Wesentlichen starre Hülle oder Manschette bildet. Für eine möglichst universelle Fixierung am Körperteil sind Befestigungsgurte in Umfangsrichtung des Körperteils angeordnet und mit üblichen einstellbaren Verbindungsbeschlägen versehen. Vakuummatratzen und -schienen sind hauptsächlich für eine horizontale Einsatzlage geeignet. Bei Verwendung in einer nicht horizontalen Lage, beispielsweise bei sitzenden Verletzten, besteht die Gefahr, daß die Füllung in einem höheren Bereich eine dünnere Schicht bildet oder sogar fehlt, weil sie sich während der Handhabung von oben nach unten verlagert hat. Es wurde daher vorgeschlagen (US 5,154,185 A) den Innenraum durch horizontale Trennwände in schmälere Kammern zu unterteilen, wobei die Trennwände Durchbrechungen aufweisen, die gerade so groß sind, daß Teile der Füllung nur händisch hindurchgedrückt werden können.

Aus der WO 99/16392 A, der als nächster Stand der Technik angesehen wird, ist eine Einrichtung der eingangs genannten Art bekannt, die ebenfalls für einen aufrechten Transport eines Verletzten geeignet ist, wobei ein Oberkörperstützkissen und ein Kopfstützkissen an einem versteifenden Rahmen befestigbar sind. Die Kissen bestehen jeweils aus zwei luftdichten Folien, die entlang ihrer Ränder und an drei Stellen im Inneren miteinander verbunden sind. Die inneren Verbindungen behindern die freie Bewegung des Granulats, sodass es trotz der aufrechten Verwendung im Wesentlichen an Ort und Stelle bleibt.

Abgesehen von durch die inneren Verbindungen verursachten Erschwernisse bei der Herstellung der Stützeinrichtungen ergeben sich zwangsläufig überall dort Bereiche ohne Granulat, wo die beiden Folien direkt miteinander verbunden sind und somit auch Bereiche, in denen trotz des Vakuums die Aussteifung der Stützeinrichtung mangelhaft ist und eine ungünstige Beweglichkeit erhalten bleibt.

In der WO 01/30280 wurde eine Lösung vorgeschlagen, in der das Granulat in Kammern unterteilte Einsatzelemente eingefüllt wurden, die aus einem luftdurchlässigen Material gefertigt sind, und nach der Füllung in das Folienelement eingesetzt werden. Dabei kann auch eine annähernd gleichmäßige Granulatschicht in der gesamten Stützeinrichtung erzielt werden, wenn zwei derartige Einsatzelemente innerhalb der Folien versetzt übereinander liegend angeordnet werden.

Die Erfindung hat es sich nun zur Aufgabe gestellt, einen praktikablen Kompromiss zu finden, der eine einfache, Material sparende Herstellung, eine zufriedenstellende, vollflächige Steifigkeit des evakuierten Stützelementes und eine rasche und den Erfordernissen entsprechende Anpassbarkeit an die Gegebenheiten darstellt.

Erfindungsgemäß wird ein derartiger Kompromiss dadurch erreicht, dass die inneren Verbindungen in einem Vierpunktraster angeordnet sind.

Es hat sich gezeigt, dass Verbindungen, die in einem Vierpunktraster über das gesamte Folienelement verteilt werden, aufgrund der Reibung zwischen den Partikeln des Granulats völlig ausreichend sind, um eine annähernd gleichmäßige Schichtdicke auch bei lotrechter Anordnung zu bewahren, wobei aber eine händische Verlagerung von Partikeln zur Anpassung an den Einsatzort sich verhältnismäßig leicht bewerkstelligen lässt. Die Herstellung der punktförmigen Verbindungen kann bei den üblicherweise verwendeten schweißbaren Kunststofffolien von außen erfolgen, und die Befüllung mit Granulat lässt sich erfindungsgemäß in einfacher Weise dadurch erzielen, dass in jeden Kanal eine Fülllanze eingeschoben und durch die Fülllanzen Granulat zugeführt wird, wobei die Fülllanzen entsprechend dem Füllfortschritt wieder herausgezogen werden, und dass schließlich die Folien am offenen oberen Querrand verbunden werden.

Da die Fülllanzen bzw. -rohre in einer Reihe nebeneinander von einem mit Granulat gefüllten Vorratsbehälter nach unten abstehen, werden bevorzugt die zu füllenden Folienelemente von unten auf die Fülllanzen aufgeschoben und mit fortschreitender Füllung wieder abgesenkt.

Die im Vierpunktraster angeordneten Verbindungen weisen natürlich auch die eingangs erwähnten Nachteile auf, nämlich, dass an jeder Verbindungsstelle kein Granulat liegen kann und dass die Verbindungsstellen in jeder Reihe oder Spalte eine linienförmige Schwachstelle darstellen, entlang der die Steifheit nach dem Evakuieren geringer ist als in den übrigen Bereichen. Aufgrund der örtlichen Beschränktheit der punktförmigen Verbindungen und des durch die einander kreuzenden Kanäle relativ großen Abstands zwischen den Verbindungsstellen bleibt aber für die meisten Anwendungsfälle eine ausreichende Festigkeit des evakuierten Folienelementes erhalten. Die zwischen den Verbindungsstellen liegenden Kanäle weisen beispielsweise eine Breite von ca. 10 cm und eine Dicke von ca. 3 cm auf, und die Füllung besteht vor allem aus einem Granulat aus geschäumtem Polystyrol mit einem Gewicht zwischen 20 kg/m³ und 70 kg/m³, insbesondere von 60 kg/m³, wobei der mittlere Durchmesser der einzelnen Partikel zwischen 0,4 mm und 5 mm, insbesondere zwischen 0,5 mm und 2 mm ist. Eine bevorzugte Ausführung der Verbindung sieht dabei vor, dass das Vierpunktraster schräg zu den Kanälen ist, wobei die vier Punkte insbesondere jeweils an den Ecken eines Quadrats liegen. Der Abstand der Verbindungspunkte in Längs- und Querrichtung erhöht sich dadurch auf die Länge der Diagonalen, und die Stabilität ist in beiden Richtungen gut.

Nachstehend wird nun die Erfindung an Hand der Figuren der beiliegenden Zeichnung näher beschrieben, ohne darauf beschränkt zu sein. Es zeigen:
- Fig. 1: eine schematische Ansicht eines rechteckigen Folienelementes,
- Fig. 2: einen Schnitt nach der Linie II-II der Fig. 1 und
- Fig. 3: eine schematische Darstellung der Füllung des Folienelementes von Fig. 1 mit Granulat.

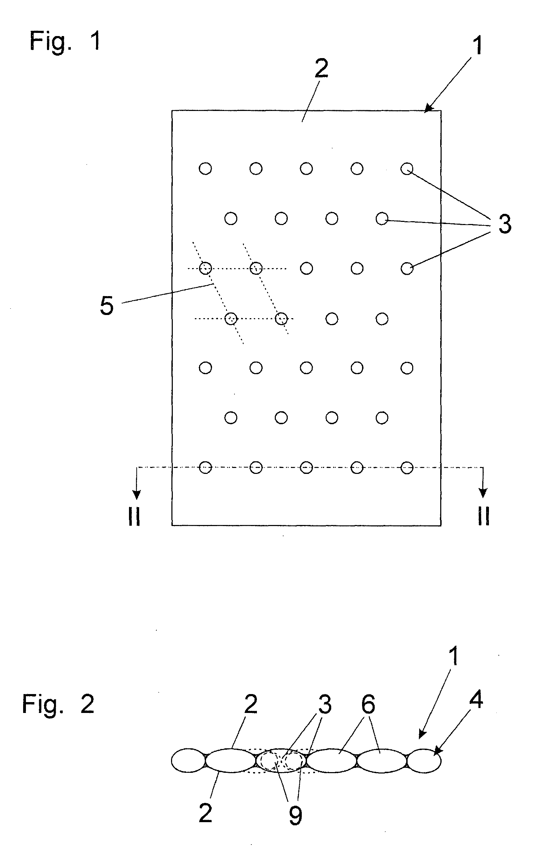

Ein Folienelement 1, das je nach Größe und Formgebung zur Immobilisierung und Stabilisierung eines verletzten Körperteils, beispielsweise eines gebrochenen Armes oder Beines, oder einer verletzten Person geeignet ist, weist zwei Folien 2 aus einem luftdichten Kunststoff, beispielsweise aus einem Polyurethan, auf, die entlang des Randes umlaufend und an Stellen 3 im Inneren miteinander verbunden, insbesondere verschweißt sind. Der Innenraum 4 enthält ein Granulat 7 aus einem leichten Kunststoff, insbesondere aus einem geschäumten Polystyrol, wobei der Durchmesser der einzelnen Kugeln insbesondere zwischen 0,5 und 2 mm liegt. Eine der beiden Folien 2 ist mit einem nicht gezeigten Ventil versehen, über das die im Innenraum 4 eingeschlossene Luft abgesaugt werden kann, wodurch das lose eingefüllte und im Innenraum 4 durch äußere Einwirkung verschiebbare bzw. verlagerbare Granulat 7 in eine dichte Packung überführt wird, die das Folienelement 1 ausreichend versteift, um eine Bewegung des verletzten Körpers bzw. Körperteils zu unterbinden. Da das Folienelement 1 nicht nur in horizontaler Lage eingesetzt werden kann, sind die Verbindungsstellen 3 im Inneren so angeordnet, dass das Granulat auch beim Anheben des Folienelementes 1 bzw. bei Verwendung in vertikaler Lage, etwa bei der Oberkörperstabilisierung eines sitzenden Verletzten, nicht nach unten rutschen kann, sondern annähernd gleichmäßig verteilt bleibt. Die Anordnung der Verbindungsstellen 3 folgt dabei einem Vierpunktraster, wie aus den in Fig. 1 strichliert eingezeichneten Rasterlinien 5 ersichtlich ist, wobei die Rasterlinien 5 schräg zu den Seitenwänden des Folienelementes 1 verlaufend dargestellt sind. Die Rasterlinien 5 können aber ebenso auch parallel zu den Seitenrändern sein. Der Abstand zwischen je zwei Verbindungsstellen 3 ist bevorzugt gleich groß, das heißt, das Raster ist quadratisch, gegebenenfalls rhombisch, und beträgt ca. 10 cm. Die Maße und Anordnung können etwas variieren, jedoch sind sie in jedem Fall so, dass in Längsrichtung des Folienelementes durchgehende, parallele Kanäle 6 verbleiben. Ein derartiges Folienelement kann wie folgt hergestellt werden:

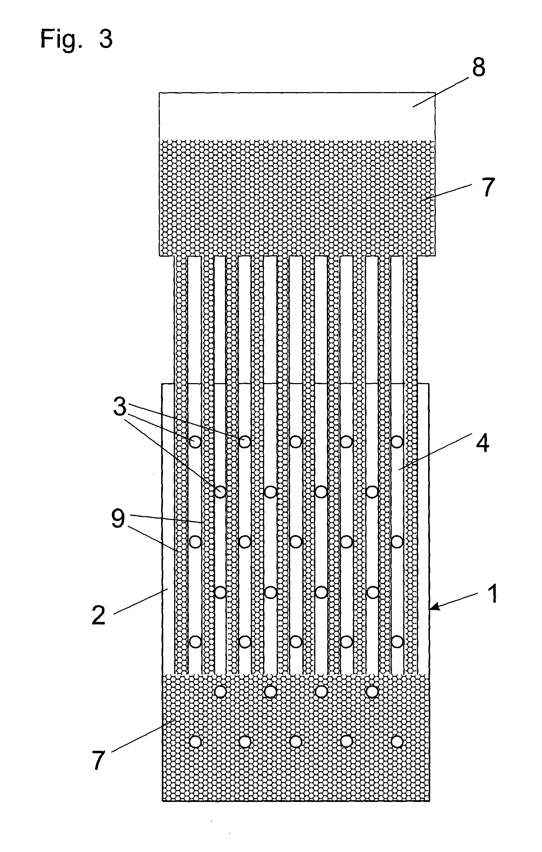

Zwei in der passenden Größe zugeschnittene Folien 2 werden an drei Seitenrändern und an den durch das Vierpunktraster vorgegebenen Verbindungsstellen 3 im Inneren miteinander verschweißt, sodass ein oben offenes sackähnliches Gebilde entsteht. Wie aus Fig. 3 ersichtlich, werden in die Kanäle 2 des oben offenen Folienelementes 1 zueinander parallele Fülllanzen 9 bzw. Füllrohre eingeführt, die von der Unterseite eines Granulat 7 beinhaltenden Behälters 8 ausgehen. Werden die nicht gezeigten Bodenauslässe des Behälters 8 geöffnet, rieselt das Granulat 7 durch jede Füllzone 9 nach unten in das Folienelement 1, das langsam nach unten abgesenkt wird, und sich dadurch mit dem Granulat 7 füllt. Anschließend werden die Folien 2 auch entlang des oberen Randes verbunden. Wie aus Fig. 3 ersichtlich, können die Fülllanzen 9 über die gesamte Höhe des Folienelementes 1 die Verbindungsstellen 3 passieren, und die Füllung verteilt sich beim Absenken des Folienelementes in die zwischen den Kanälen 6 liegenden Freiräume, die direkt zwischen zwei übereinander liegenden Verbindungsstellen 3 liegen.

## Patentansprüche

1. Einrichtung zur Stützung und Stabilisierung eines Verletzten, mit einem flexiblen, am Verletzten festlegbaren Folienelement (1), das zwei Folien (2) und einen Granulat (9) enthaltenden, evakuierbaren Innenraum (4) aufweist, wobei die beiden Folien (2) entlang des Randes und an Stellen (3) im Inneren im Wesentlichen punktförmig so miteinander verbunden sind, dass Kanäle (6) parallel zu mindestens einem Seitenrand verbleiben, **dadurch gekennzeichnet, dass** die inneren Verbindungen (3) in einem Vierpunktraster (5) angeordnet sind.

2. Einrichtung nach Anspruch 1, **dadurch gekennzeichnet, dass** das Vierpunktraster (5) schräg zu den Kanälen (6) ist.

3. Einrichtung nach Anspruch 2, **dadurch gekennzeichnet, dass** das Vierpunktraster (5) gleiche Seitenlängen aufweist.

4. Einrichtung nach einem der Ansprüche 1 bis 3, **dadurch gekennzeichnet, dass** die Anordnung der inneren Verbindungen mehrere Zeilen und Spalten mit jeweils mehr als zwei Verbindungsstellen (3) umfasst.

5. Einrichtung nach einem der Ansprüche 1 bis 4, **dadurch gekennzeichnet, dass** das Granulat (9) aus geschäumtem Polystyrol mit einem Gewicht zwischen 20 kg/m³ und 70 kg/m³ besteht.

6. Einrichtung nach einem der Ansprüche 1 bis 4, **dadurch gekennzeichnet, dass** das Granulat (9) aus geschäumtem Polystyrol mit einem Durchmesser zwischen 0,4 mm und 5 mm besteht.

7. Einrichtung nach einem der Ansprüche 1 bis 6, **dadurch gekennzeichnet, dass** das Granulat (9) aus geschäumtem Polystyrol mit einem Gewicht von 60 kg/m³ und einem Durchmesser zwischen 0,5 mm und 2 mm besteht.

8. Einrichtung nach einem der Ansprüche 1 bis 7, **dadurch gekennzeichnet, dass** die Kanäle eine Breite von ca. 10 cm und eine Dicke von ca. 3 cm aufweisen.

9. Verfahren zur Herstellung einer Einrichtung nach einem der Ansprüche 1 bis 8, **dadurch gekennzeichnet, dass** zwei Folien (2) unten, seitlich und punktuell im Inneren so miteinander verbunden werden, dass zueinander parallele, nach oben offene Kanäle (6) verbleiben, dass in jeden Kanal (6) eine Fülllanze (9) eingeschoben und durch die Fülllanzen (9) Granulat (7) zugeführt wird, wobei die Fülllanzen (9) entsprechend dem Füllfortschritt wieder herausgezogen werden, und dass schließlich die Folien (2) am offenen oberen Querrand verbunden werden.

## Claims

1. A device for supporting and stabilising an injured person comprising a flexible film element (1) which can be fixed to the injured person and which has two films (2) and an evacuatable interior (4) containing a granular material (9), wherein the two films (2) are connected together in substantially point form along the edge and at locations (3) in the interior in such a way that passages (6) parallel to at least one side edge remain **characterised in that** the internal connections (3) are arranged in a four-point grid (5).

2. A device according to claim 1 **characterised in that** the four-point grid (5) is inclined relative to the passages (6).

3. A device according to claim 2 **characterised in that** the four-point grid (5) has equal side lengths.

4. A device according to one of claims 1 to 3 **characterised in that** the arrangement of the internal connections includes a plurality of rows and columns respectively having more than two connecting locations (3).

5. A device according to one of claims 1 to 4 **characterised in that** the granular material (9) comprises foamed polystyrene of a weight of between 20 kg/m³ and 70 kg/m³.

6. A device according to one of claims 1 to 4 **characterised in that** the granular material (9) comprises foamed polystyrene of a diameter of between 0.4 mm and 5 mm.

7. A device according to one of claims 1 to 6 **characterised in that** the granular material (9) comprises foamed polystyrene of a weight of 60 kg/m³ and of a diameter of between 0.5 mm and 2 mm.

8. A device according to one of claims 1 to 7 **characterised in that** the passages are of a width of about 10 cm and a thickness of about 3 cm.

9. A process for the production of a device according to one of claims 1 to 8 **characterised in that** two films (2) are connected together underneath, laterally and in point relationship in the interior in such a way that mutually parallel, upwardly open passages (6) remain, that a filling lance (9) is inserted into each passage (6) and granular material (7) is supplied through the filling lances (9), wherein the filling lances (9) are pulled out again corresponding to the progression in the filling operation, and that finally the films (2) are joined at the open upper transverse edge.

## Revendications

1. Dispositif de soutien et de stabilisation d'un blessé, comprenant un élément sous forme de feuilles (1) flexibles pouvant être fixé sur le blessé et comportant deux feuilles (2) et un espace intérieur (4) contenant des granulés (9) où un vide peut être généré, les deux feuilles (2) étant assemblées l'une à l'autre le long de leur bord et sensiblement par des points à des emplacements (3) à l'intérieur, de telle manière que des canaux (6) restent parallèles à au moins un bord latéral, **caractérisé en ce que** les jonctions (3) intérieures sont disposées en formant une trame à quatre points (5).

2. Dispositif selon la revendication 1, **caractérisé en ce que** la trame à quatre points (5) est oblique par rapport aux canaux (6).

3. Dispositif selon la revendication 2, **caractérisé en ce que** la trame à quatre points (5) a des côtés de longueur égale.

4. Dispositif selon l'une des revendications 1 à 3, **caractérisé en ce que** l'agencement des jonctions intérieures comprend plusieurs rangées et colonnes comprenant chacune plus de deux emplacements de jonction (3).

5. Dispositif selon l'une des revendications 1 à 4, **caractérisé en ce que** les granulés (9) sont en polystyrène moussé avec un poids compris entre 20 kg/m³ et 70 kg/m³.

6. Dispositif selon l'une des revendications 1 à 4, **caractérisé en ce que** les granulés (9) sont en polystyrène moussé avec un diamètre compris entre 0,4 mm et 5 mm.

7. Dispositif selon l'une des revendications 1 à 6, **caractérisé en ce que** les granulés (9) sont en polystyrène moussé avec un poids de 60 kg/m³ et un diamètre compris entre 0,5 mm et 2 mm.

8. Dispositif selon l'une des revendications 1 à 7, **caractérisé en ce que** les canaux ont une largeur de 10 cm environ et une épaisseur de 3 cm environ.

9. Procédé de fabrication d'un dispositif selon l'une des revendications 1 à 8, **caractérisé en ce que** deux feuilles (2) sont assemblées l'une à l'autre sur le bas, sur les côtés et par des points à l'intérieur, de telle manière que des canaux (6) parallèles ouverts sur le haut sont formés, qu'une lance de remplissage (9) puisse être introduite dans chaque canal (6) et que des granulés (7) soient refoulés par les lances de remplissage (9), les lances de remplissage (9) étant retirées ensuite en fonction de l'état du remplissage, et que les feuilles (2) soient finalement assemblées sur leur bord transversal supérieur ouvert.
